# EUROPEAN PATENT SPECIFICATION

(11) **EP 0 295 043 B1**
(45) Date of publication and mention of the grant of the patent: **26.05.1993**
(21) Application number: 88305166.6
(22) Date of filing: 07.06.1988
(51) Int. Cl.: A61K 39/12, A61K 39/20, A61K 39/165

(54) **Stabilized live attenuated vaccine and its production**
Stabilisierter attenuierter lebender Impfstoff und seine Herstellung
Vaccin vivant atténué stabilisé et sa production

(30) Priority: 08.06.1987 JP 142465/87
(43) Date of publication of application: 14.12.1988
(73) Proprietor: THE KITASATO INSTITUTE, Minato-ku Tokyo (JP)
(72) Inventor: Makino, Satoshi, Yokohama-shi Kanagawa-ken (JP); Sasaki,Keiko, Kawasaki-shi Kanagawa-ken (JP); Nakagawa, Masaharu, Nerima-ku Tokyo (JP)
(74) Representative: Woods, Geoffrey Corlett

(56) References cited:
- EP-A- 0 028 563
- EP-A- 0 065 905
- EP-A- 0 230 265
- FR-A- 2 424 031

## Description

This invention relates to a stabilized live attenuated vaccine and its production.

A titer of live attenuated vaccine is in general temperature unstable. A vaccine preparation is therefore supplied as a low-temperature freezed product or a lyophilized product. To stabilize the vaccine further a chemical stabilizing agent may be added to the vaccine solution. Examples of chemical stabilizing agents are human albumin, gelatin hydrolyzate, sugar alcohols, amino acids and other non-toxic substances. Known examples are a preparation consisting of 5 W/V% of a basic amino acid such as arginine, lysine or histidine, or 5 W/V% of a sugar alcohol such as saccharose, adnitol or sorbitol (Japan. Patent Appln. No. 45-1887); a preparation comprising a mixture of 5 to 10 W/V% peptone, 3 W/V% arginine or lysine and 5 W/V% saccharose (Japan. Pat. Unexam. Publ. No. 50-2225), a preparation comprising 4 W/V% lactose and 2 W/V% sorbitol or 0.005M to 0.05M of at least one amino acid in a phosphate-buffer solution containing Ca⁺⁺ and Mg⁺⁺ (Japan. Pat. Appln. No. 57-81338), a preparation consisting of 5 W/V% lactose, 1.5 W/V% D-sorbitol, 0.3 W/V% dextran 70, 0.048% potassium glutamate, 0.0625 W/V% disodium phosphate, 0.026 W/V% potassium phosphate, 0.3 W/V% gelatin and 0.25 W/V% human albumin (Japan. Pat. Appln. No. 55-80465), and a preparation comprising a vaccine solution which has been acidified to pH 6.0 to 6.5 prior to lyophilization by adding a phosphate buffer solution containing a stabilizing agent comprising gelatin partial hydrolysate M.W. approx. 3,000, sorbitol, saccharose, lactose, maltose, L-glutamate and L-arginine (Japan. Pat. Unexam. Publ. No. 57-114527). EP-A-0 028 563 discloses a vaccine stabilizing composition in buffered solution containing gelatin, a monosaccharide (e.g. sorbitol, lactose) or disaccharide, glutamic acid (sodium salt) and arginine.

Nowadays, vaccines have to be distributed to tropical countries where there are no cold-chain distribution systems. In these areas, the vaccine preparations which are supplied must have heat stability in high temperatures. The above stabilizing agents are not suitable for use in such conditions.

We have found that a live attenuated vaccine shows strong thermostability when lactose, D-sorbitol, saccharose, gelatin hydrolysate and sodium glutamate at specific ratio in concentration were added to a vaccine virus suspension comprising medium-199 for cell culture (Morgan et al. Proc. Soc. Exp. Biol. Med.,73: 1-8, 1950) (hereinafter referred to as medium - 199). Each component is known as a stabilizing agent for a live attenuated vaccine, however it has not previously been known that a combination thereof at a specific concentration ratio exhibits unexpected thermostability.

The present invention provides a vaccine, which comprises at least one live attenuated plain vaccine selected from measles, mumps or rubella virus, for example which has been grown in a medium-199 for cell culture, and, as a stabilizing agent, 2.5 to 5 W/V% lactose, 2.5 to 5 W/V% saccharose, 1.8 to 2 W/V% D-sorbitol, about 0.1 W/V% sodium glutamate and 2 to 3 W/V% gelatin hydrolysate having a molecular weight of about 35,000.

The present invention also provides a process for preparing a vaccine which comprises adding at least one live attenuated plain vaccine selected from measles, mumps or rubella virus, lactose, saccharose, D-sorbitol, sodium glutamate and gelatin hydrolysate having a molecular weight of about 35,000, each being added individually or in any combination thereof, as a stabilizing agent, to provide a final concentration of 2.5 to 5 W/V% lactose, 2.5 to 5 W/V% saccharose, 1.8 to 2 W/V% D-sorbitol, about 0.1 W/V% sodium glutamate and 2 to 3 W/V% said gelatin hydrolysate.

The stabilizing agent may be added directly to the virus in the medium in which the virus is cultured. The virus may be cultured in medium 199. The stabilizing agent may be dissolved in medium 199 at the desired concentration and aseptically filtered. The vaccine may be lyophilized and then reconstituted. In the present invention it is not required to adjust the pH of the vaccine solution to less than neutral but only to add each sterilized component. A further advantage of the present invention is that the vaccine does not contain human albumin which provides a superior stabilization effect; the additives used are more safe. Therefore the adverse effects of immunological preparations caused by the carrier or vehicle can be reduced.

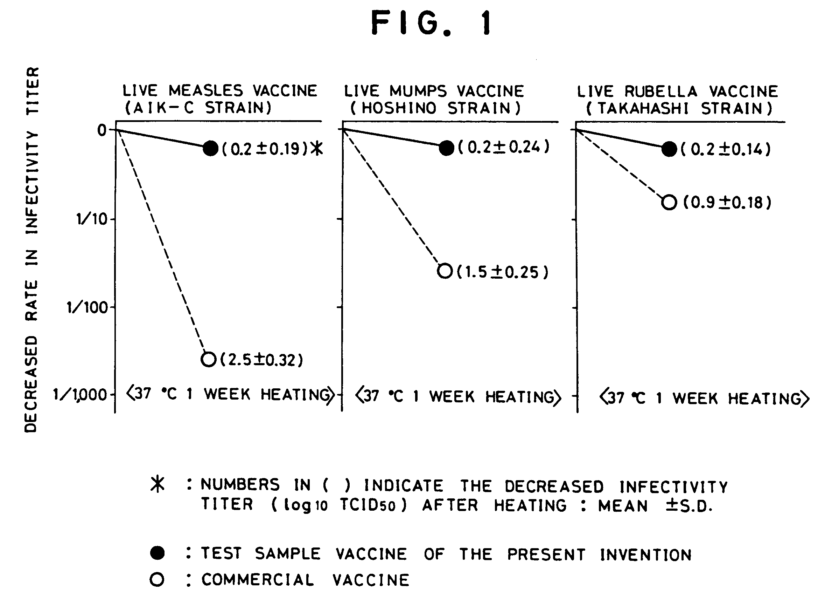

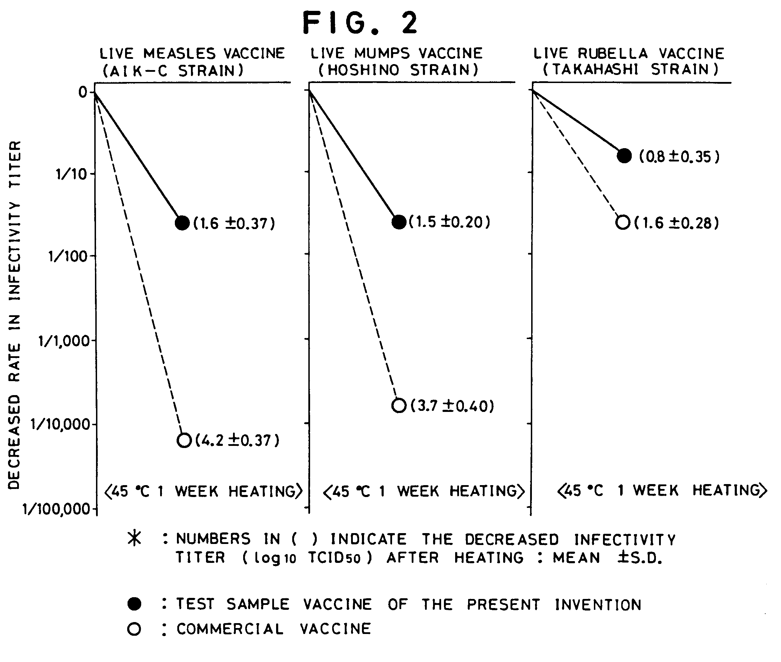

The thermostability of the vaccine of the present invention can be observed at virus suspension (Table 1), and is more apparent in the lyophilized product (Table 2). The thermostability of the lyophilized live attenuated vaccine product of the present invention is superior than that of the known vaccine preparation containing a conventional stabilizing agent. For example when a measles vaccine preparation containing a conventional stabilizing agent consisting of 5 W/V% lactose, 0.1 W/V% sodium glutamate and 0.2 W/V% gelatin hydrolysate is kept at 37° for one week, its titer decreases to 1/100 to 1/500 with loss of immunogenicity. In contrast the vaccine of the present invention loses its titer at most up to 1/3 without losing immunogenicity as compared with a titer of an original vaccine preparation. As shown in Figs. 1 and 2, the thermostability of mumps and rubella vaccines of the present invention is also superior than the commercial vaccine preparation containing a conventional stabilizing agent. A live attenuated measles vaccine of the present invention can be used for the worldwide project for prophylaxis of measles promoted by WHO, including in countries having no cold-chain distribution systems.

Figs. 1 and 2 show the heat-stability of vaccines of the present invention and of commercially available vaccines.

The following Example further illustrates the present invention.

### EXAMPLE

5 W/V% Lactose, 5 W/V% saccharose, 1.8 W/V% D-sorbitol, 0.1 W/V% sodium glutamate, and 2 W/V% gelatin hydrolysate having a molecular weight of about 35,000 [Behringwerke AG, West Germany, gelatin hydrolysate powder dissolved in medium-199 for raw material of "HEMACEL" (Trade name, gelatin hydrolysate for liquid transfusion)] were added to a measles vaccine virus suspension in medium-199 to prepare a bulk vaccine (2 batches). The bulk vaccine was lyophilized and finally 5 lots of vaccine preparation were produced. A comparative accelerated heat-stability test with this vaccine and a commercial vaccine lyophilized product (4 lots) was performed.

Live attenuated mumps or rubella vaccines of the present invention were prepared, each in 2 batches, and the vaccine solutions were lyophilized to prepare 4 lots of each product. A comparative accelerated heat-stability test with commercial vaccine lyophilized products (each 2 lots) was carried out. As shown in Table 2 and Figs. 1 and 2, the decrease in titer of the lyophilized live attenuated measles, mumps and rubella vaccines of the present invention, after keeping at 37°C and at 45°C for one week, is significantly less than that of the commercially available conventional vaccines.

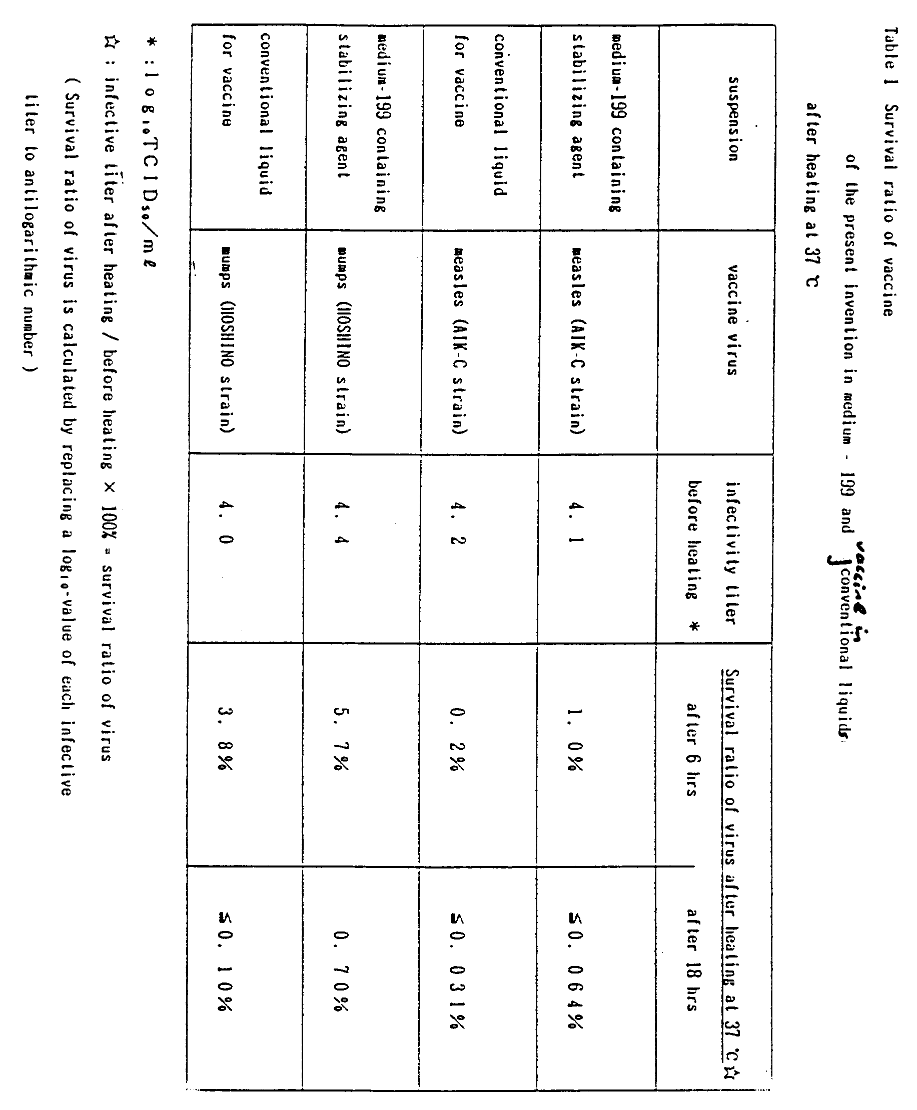

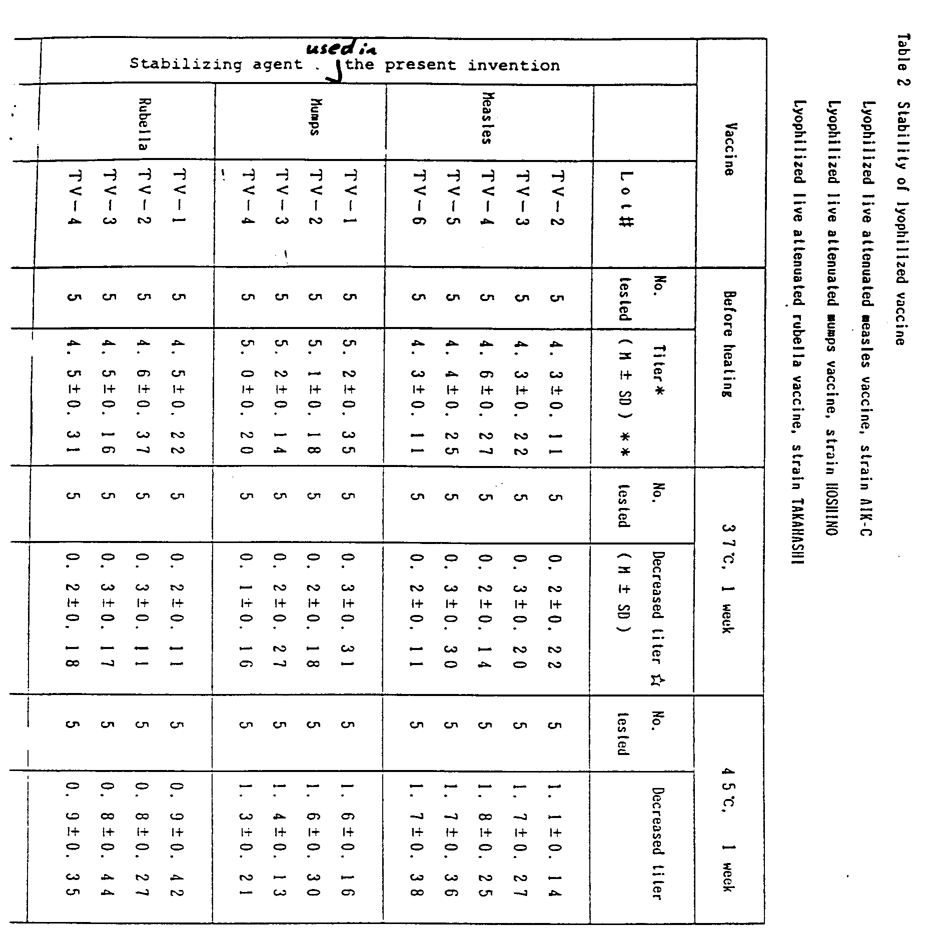

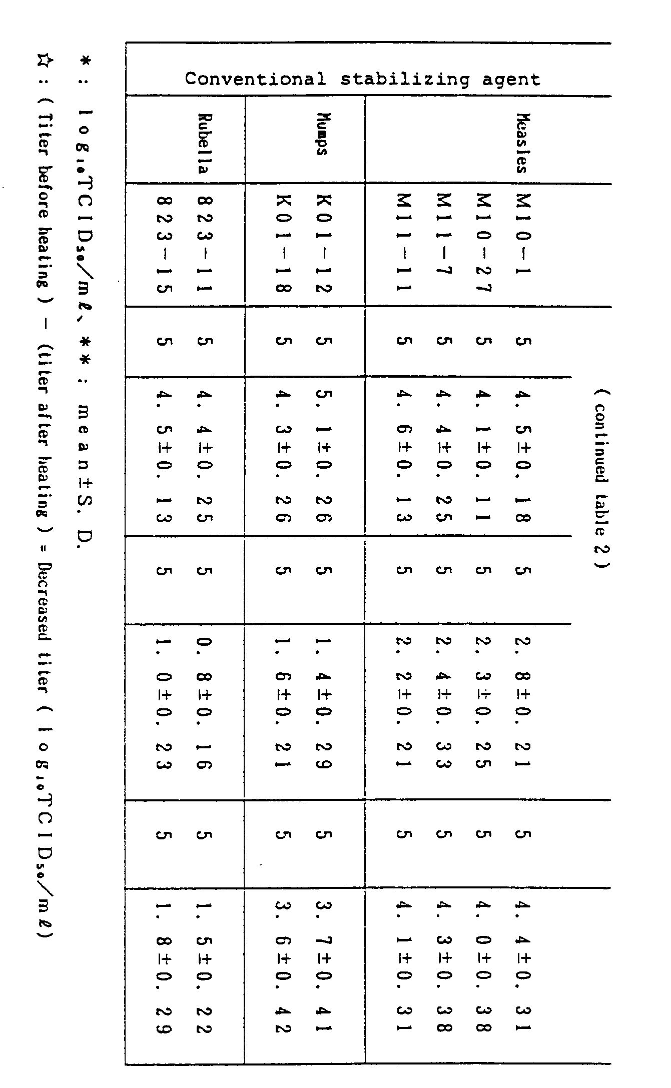

## Claims (Claims for the following Contracting State(s): AT, CH, DE, FR, GB, IT, LI, LU, NL, SE)

1. A vaccine, which comprises at least one live attenuated plain virus selected from measles, mumps or rubella virus and, as a stabilizing agent, 2.5 to 5 W/V% lactose, 2.5 to 5 W/V% saccharose, 1.8 to 2 W/V% D-sorbitol, about 0.1 W/V% sodium glutamate and 2 to 3 W/V% gelatin hydrolysate having a molecular weight of about 35,000.

2. A process for preparing a vaccine, which comprises adding to at least one live attenuated plain virus selected from measles, mumps or rubella virus, lactose, saccharose, D-sorbitol, sodium glutamate and gelatin hydrolysate having a molecular weight of about 35,000, each being added individually or in any combination thereof, as a stabilizing agent, to provide a final concentration of 2.5 to 5 W/V% lactose, 2.5 to 5 W/V% saccharose, 1.8 to 2 W/V% D-sorbitol, about 0.1 W/V% sodium glutamate and 2 to 3 W/V% said gelatin hydrolysate.

3. A process according to claim 2 wherein the stabilizing agent is added directly to the virus in the medium in which the virus is cultured.

4. A process according to claim 2 or 3 wherein the virus has been cultured in medium-199.

5. A process according to claim 2, 3 or 4 which further comprises lyophilizing said vaccine.

6. A process according to claim 5 which further comprises reconstituting the lyophilized vaccine.

## Claims (Claims for the following Contracting State(s): ES, GR)

1. A process for preparing a vaccine, which comprises adding to at least one live attenuated plain virus selected from measles, mumps or rubella virus, lactose, saccharose, D-sorbitol, sodium glutamate and gelatin hydrolysate having a molecular weight of about 35,000, each being added individually or in any combination thereof, as a stabilizing agent, to provide a final concentration of 2.5 to 5 W/V% lactose, 2.5 to 5 W/V% saccharose, 1.8 to 2 W/V% D-sorbitol, about 0.1 W/V% sodium glutamate and 2 to 3 W/V% said gelatin hydrolysate.

2. A process according to claim 1 wherein the stabilizing agent is added directly to the virus in the medium in which the virus is cultured.

3. A process according to claim 1 or 2 wherein the virus has been cultured in medium-199.

4. A process according to claim 1, 2 or 3 which further comprises lyophilizing said vaccine.

5. A process according to claim 4 which further comprises reconstituting the lyophilized vaccine.

## Patentansprüche (Patentansprüche für folgende(n) Vertragsstaat(en): AT, CH, DE, FR, GB, IT, LI, LU, NL, SE)

1. Impfstoff, der mindestens einen attenuierten lebenden nackten Virus umfaßt, ausgewählt aus Masern-, Mumps- oder Röteln-Virus und als Stabilisierungsmittel 2.5 bis 5 G/V% Lactose, 2.5 bis 5 G/V% Saccharose, 1.8 bis 2 G/V% D-Sorbit, ca. 0.1 G/V% Natriumglutamat und 2 bis 3 G/V % Gelatine-Hydrolysat mit einem Molekulargewicht von ca. 35000.

2. Verfahren zur Herstellung eines Impfstoffes, das die Zugabe von Lactose, Saccharose, D-Sorbit, Natriumglutamat und Gelatine-Hydrolysat mit einem Molekulargewicht von etwa 35000 zu mindestens einem attentuierten lebenden nackten Virus, ausgewählt aus Masern-, Mumps- oder Röteln-Virus, umfaßt, wobei jeder Bestandteil individuell oder in irgendeiner Kombination miteinander als Stabilisierungsmittel zugegeben wird, um eine Endkonzentration von 2.5 bis 5 G/V% Lactose, 2.5 bis 5 G/V Saccharose, 1.8 bis 2 G/V% D-Sorbit, ca. 0.1 G/V% Natriumglutamat und 2 bis 3 G/V% des Gelatine-Hydrolysates zu ergeben.

3. Verfahren nach Anspruch 2, dadurch gekennzeichnet, daß das Stabilisierungsmittel direkt zum Virus in dem Medium, in dem der Virus kultiviert wird, zugegeben wird.

4. Verfahren nach Anspruch 1 oder 2, dadurch gekennzeichnet, daß der Virus im Medium-199 kultiviert wurde.

5. Verfahren nach Anspruch 2, 3 oder 4, dadurch gekennzeichnet, daß es ferner die Lyophilisation des Impfstoffes umfaßt.

6. Verfahren nach Anspruch 5, dadurch gekennzeichnet, daß es ferner die Rekonstitution des lyophilisierten Impfstoffes umfaßt.

## Patentansprüche (Patentansprüche für folgende(n) Vertragsstaat(en): ES, GR)

1. Verfahren zur Herstellung eines Impfstoffs, das die Zugabe von Lactose, Saccharose, D-Sorbit, Natriumglutamat und Gelatine-Hydrolysat mit einem Molekulargewicht von ca. 35000 zu mindestens einem attenuierten lebenden nackten Virus, ausgewählt aus Masern-, Mumps- oder Röteln-Virus umfaßt, wobei jeder Bestandteil individuell oder in irgendeiner Kombination miteinander als Stabilisierungsmittel zugegeben wird, um eine Endkonzentration von 2.5 bis 5 G/V% Lactose, 2.5 bis 5 G/V% Saccharose, 1.8 bis 2 G/V% D-Sorbit, ca. 0.1 G/V% Natriumglutamat und 2 bis 3 G/V% des Gelatine-Hydrolysats zu ergeben.

2. Verfahren nach Anspruch 1, dadurch gekennzeichnet, daß das Stabilisierungsmittel direkt zum Virus in dem Medium, in dem der Virus kultiviert wird, zugegeben wird.

3. Verfahren nach Anspruch 1 oder 2, dadurch gekennzeichnet, daß der Virus im Medium-199 kultiviert wurde.

4. Verfahren nach Anspruch 1, 2 oder 3, dadurch gekennzeichnet, daß es ferner die Lyophilisation des Impfstoffes umfaßt.

5. Verfahren nach Anspruch 4, dadurch gekennzeichnet, daß es ferner die Rekonstitution des lyophilisierten Impfstoffes umfaßt.

## Revendications (Revendications pour l'(les) Etat(s) contractant(s) suivant(s): AT, CH, DE, FR, GB, IT, LI, LU, NL, SE)

1. Vaccin, qui comprend au moins un simple virus vivant atténué choisi parmi les virus de la rougeole, des oreillons ou de la rubéole, et, comme agent de stabilisation, 2,5 à 5% en poids/volume de lactose, 2,5 à 5% en poids/volume de saccharose, 1,8 à 2% en poids/volume de D-sorbitol, environ 0,1% en poids/volume de glutamate de sodium et 2 à 3% en poids/volume d'hydrolysat de gélatine ayant un poids moléculaire d'environ 35 000.

2. Procédé pour préparer un vaccin, dans lequel on ajoute à au moins un simple virus vivant atténué choisi parmi les virus de la rougeole, des oreillons ou de la rubéole, du lactose, du saccharose, du D-sorbitol, du glutamate de sodium et un hydrolysat de gélatine ayant un poids moléculaire d'environ 35 000, chaque composant étant ajouté individuellement ou en toute combinaison, comme agent de stabilisation, pour obtenir une concentration finale, en poids/volume, de 2,5 à 5% de lactose, 2,5 à 5% de saccharose, 1,8 à 2% de D-sorbitol, environ 0,1% de glutamate de sodium et 2 à 3% de cet hydrolysat de gélatine.

3. Procédé selon la revendication 2, dans lequel l'agent de stabilisation est ajouté directement au virus dans le milieu dans lequel le virus est cultivé.

4. Procédé selon la revendication 2 ou la revendication 3, dans lequel le virus a été cultivé dans le milieu-199.

5. Procédé selon l'une des revendications 2, 3 ou 4, qui consiste en outre à lyophiliser le vaccin.

6. Procédé selon la revendication 5, qui consiste en outre à reconstituer le vaccin lyophilisé.

## Revendications (Revendications pour l'(les) Etat(s) contractant(s) suivant(s): ES, GR)

1. Procédé pour préparer un vaccin, dans lequel on ajoute à au moins un simple virus vivant atténué choisi parmi les virus de la rougeole, des oreillons ou de la rubéole, du lactose, du saccharose, du D-sorbitol, du glutamate de sodium et de l'hydrolysat de gélatine ayant un poids moléculaire d'environ 35 000, chacun de ces composants étant ajouté individuellement ou en toute combinaison, comme agent de stabilisation, pour obtenir une concentration finale, en poids/volume, de 2,5 à 5% de lactose, 2,5 à 5% de saccharose, 1,8 à 2% de D-sorbitol, environ 0,1% de glutamate de sodium et 2 à 3% de cet hydrolysat de gélatine.

2. Procédé selon la revendication 1, dans lequel l'agent de stabilisation est ajouté directement au virus dans le milieu dans lequel le virus est cultivé.

3. Procédé selon la revendication 1 ou la revendication 2, dans lequel le virus a été cultivé dans le milieu-199.

4. Procédé selon l'une des revendications 1, 2 ou 3, qui consiste en outre à lyophiliser le vaccin.

5. Procédé selon la revendication 4, qui consiste en outre à reconstituer le vaccin lyophilisé.
